# EUROPÄISCHE PATENTSCHRIFT

(11) **EP 0 675 101 B1**
(45) Veröffentlichungstag und Bekanntmachung des Hinweises auf die Patenterteilung: **15.04.1998**
(21) Anmeldenummer: 95108067.0
(22) Anmeldetag: 02.07.1992
(51) Int. Cl.: C07C 67/03, C07C 69/614, C07K 1/00

(54) **Umesterung und andere Umsetzungsreaktionen von Säurederivaten mit einer Amidinbase**
Transesterification and other conversion reactions of acid derivatives with an amidine base
Transestérification et autres réactions de transformation de dérivés d'acides avec une base amidine

(30) Priorität: 15.07.1991 DE 4123408; 22.07.1991 DE 4124283
(43) Veröffentlichungstag der Anmeldung: 04.10.1995
(62) Teilanmeldung aus: 92111238.9
(73) Patentinhaber: Degussa Aktiengesellschaft, 60311 Frankfurt (DE)
(72) Erfinder: Drauz, Karlheinz, Prof. Dr., D-63579 Freigericht (DE); Müller, Thomas, Dr., D-60489 Frankfurt (DE); Kottenhahn, Matthias, Dr., D-63579 Freigericht (DE); Seebach, Dieter, Prof. Dr., CH-8044 Zürich (CH); Thaler, Adrian, Dr., CH-6312 Steinhausen (CH)

(56) Entgegenhaltungen:
- EP-A- 0 110 629
- EP-A- 0 150 962

## Beschreibung

Die Erfindung betrifft ein Verfahren zur Umsetzung eines Phosphorsäure-, Phosphonsäure- oder Carbonsäurederivates mit einem Alkohol, Wasser oder NH₃ in Gegenwart einer Amidinbase und insbesondere ein Verfahren zur Umesterung, Ammonolyse oder Verseifung von Carbonsäure-, Phosphonsäure- oder Phosphorsäurederivaten und die Abspaltung von Aminosäure-, Peptid- oder Nukleinsäurederivaten von einem polymeren Träger. Die Erfindung betrifft außerdem ein Verfahren zur Verseifung eines Peptidesters in wässrigem Medium in Gegenwart einer Metallverbindung als Base. In der Literatur wurden bereits verschiedene Verfahren zur Umesterung oder Verseifung der genannten Verbindungen beschrieben. Hierbei wird z. B. unter stark sauren oder basischen Bedingungen, mit Enzymen (D. Seebach, Angew. Chem. 1990, 102, 1363), mit Titanaten (D. Seebach, B. Weidmann, L. Widler in Modern Synthetic Methods" 1983), mit KF/Kronenether (B. Lejczak, P. Kafarski, J. Szewczyk, Synthesis 1982, 412) mit Ionenaustauscherharzen (W. Pereira, V. Close, W. Patton, B. Halpfern, J. Org. Chem. 1969, 34, 2032) oder Distannoxanen (J. Otera, S. Ioka, H. Nozaki, J. Org. Chem. 1989, 54, 4013) gearbeitet. Weiterhin wurde ein Verfahren zur Umesterung bzw. Verseifung von Esterderivaten unter Verwendung der Amidinbase DBU in Lösung (EP-en 0110629, 0150962) oder polymergebunden (T. Ishikawa, Y. Ohsumi, T. Kawai, Bull. Chem. Soc. Jpn. 1990, 63, 819) beschrieben.

Aus der EP 0 110 629 A1 ist die Verwendung von Amidinbasen zur Umesterung bekannt. Das Amidin wird hier meist mit Epoxiden unterstützt. Besonders milde Bedingungen, wie sie bei der Umsetzung insbesondere optisch aktiver Biomoleküle notwendig sind, können dieser Schrift nicht entnommen werden. In allen Beispielen werden einfache, stabile Verbindungen ohne zusätzliche funktionelle Gruppen oder optischer Aktivitäten beschrieben.

Aus Int. J. Peptide Protein Res. 37, 1991, 451 - 456 sind Umesterungen unter Verwendung von Calciumacetat in Methanol bekannt. Als Substrat werden weit überwiegend nur Peptide mit C-terminalem Glycinrest eingesetzt, solche Peptide sind unempfindlich gegenüber Racemisierungen. Bei der Verwendung eines C-terminalen Alanylrestes ist die Reaktion schon inhibiert. Außerdem verlangt diese Reaktion ganz spezifische Schutzgruppen. Ein allgemeines mildes Verfahren zur Umsetzung insbesondere von Biomolekülen ist durch die Verwendung von Calciumacetat nicht gegeben.

Aus J. Org. Chem. 38 (1973), 1223 - 1225 ist die Spaltung von Carbonsäuremethylestern mit DBU bei 165°C über 48 h bekannt. Solche Bedingungen sind für die meisten Esterspaltungen zu drastisch, dies gilt insbesondere für optisch aktive Ester, die in α-Stellung zur Estergruppe ein Chiralitätszentrum besitzen.

Aus CA 114, 186011 (1991) und Tetrahedron Letters 21 (1980), 1181 - 1184 ist eine β-Eliminierung mit DBU oder DBN bekannt. Wie bei β-Eliminierungen allgemein üblich und hier auch erwähnt kann prinzipiell fast jede Base, z. B. auch Kaliumhydroxid eingesetzt werden. Allgemein verwendbare milde Reaktionsbedingungen sind hier nicht beschrieben.

Alle bisher genannten Verfahren mit Ausnahme der enzymatischen Methode benötigen im Normalfall erhöhte Temperaturen, extreme pH-Werte und/oder lange Reaktionszeiten. Für empfindliche Esterderivate, die z.B. weitere funktionelle Gruppen tragen oder ein oder mehrere chirale C-Atome besitzen, insbesondere für solche Verbindungen, die in α-Position zur Esterfunktion ein chirales C-Atom besitzen, sind die meisten der genannten Methoden aufgrund der Reaktionsbedingungen ungeeignet.

Aufgabe der vorliegenden Erfindung ist daher ein System zur Umsetzung, insbesondere Verseifung, Ammonolyse oder Umesterung von Säurederivaten und Spaltung von polymergebundenen Moleküle, das so milde Reaktionbedingungen hat, daß insbesondere optisch aktive und Biomoleküle wie Peptide, Aminosäuren oder Nukleinsäuren eingesetzt werden können.

Diese Aufgabe wird gelöst durch den kombinierten Einsatz von einer Amidinbase und einer Metallverbindung insbesondere bei der Umesterung, Verseifung oder Abspaltung polymergebundener Moleküle, wie sie z.B. bei der Merrifield-Synthese vorliegen. Bei der Verseifung von Peptidestern zeigte es sich, daß hier auch ohne die Amidinbase, einzig schon mit Lithiumhydroxid unter milden Bedingungen gespalten werden kann siehe Stammanmeldung.

Wir fanden nun überraschenderweise, daß die Verwendung einer Amidinbase in Kombination mit einem Metallsalz die Umesterung, Ammonolyse oder Verseifung der genannten Säurederivate so stark beschleunigt, daß auch empfindliche Säurederivate unter schonenden Bedingungen, d. h. tiefen Temperaturen und kurzen Reaktionszeiten erfolgreich umgesetzt werden können.

So können z.B. empfindliche Peptidester ohne Razemisierung am α-C-Atom der C-terminal positionierten Aminosäure umgeestert oder verseift werden. Dabei bleiben eventuell geschützte Seitenkettenfunktionalitäten, die keine Esterfunktion enthalten, unberührt. So war es z.B. möglich ein Heptapeptidester mit DBU/LiBr in THF/H₂O in kurzen Reaktionszeiten quantitativ und ohne Razemisierung zu verseifen. Die Verseifung war dagegen mit wässriger NaOH nicht zersetzungsfrei und führte zur Razemisierung.

Desweiteren ist es überraschenderweise mit dem erfindungsgemäßen Verfahren möglich, einfach Ester wie z.B. Methylester in komplexe Ester, wie z.B. Menthylester umzuwandeln.

Mit Hilfe neuerer Techniken werden heutzutage Peptide und Polynucleotide an Polymer-Trägern synthetisiert (Merrifield-Synthese). Die Verknüpfung mit dem Polymerträger ist dabei meist eine Ester- oder Amidbindung. Die Abspaltung vom Trägermaterial erfordert oft drastische Reaktionsbedingungen und führt in der Regel zum Verlust sämtlicher Schutzgruppen eventuell geschützter Funktionalitäten im Peptid oder Polynucleotid. So werden als gängige Abspaltungsreagentien Trifluoressigsäure/HBr/TMS-trifluormethansulfonat, HF/Anisol, NaOH/Dioxan/H₂O₂ oder Dimethylaminoethanol/Thalliumethanolat eingesetzt. Gegenüber diesen drastischen Bedingungen eignet sich die Reagentienkombination Amidinbase/Metall- und insbesondere Lithiumsalz ausgezeichnet zur Spaltung der Bindung eines Peptides oder Polynukleotides vom Polymer-Träger. Dabei können bei der Abspaltung zersetzungs- und razemisierungsfrei wahlweise Ester, Amide oder die entsprechenden freien Säuren des Peptides oder Polynukleotides erhalten werden.
Die nicht basenlabilen Schutzgruppen eventueller weiterer funktioneller Gruppen im Molekül bleiben dabei unberührt. Dabei eignet sich diese Methode insbesondere zur Freisetzung empfindlicher Moleküle sowie für die Darstellung geschützter Peptidsegmente am Polymer-Träger, die für eine weitere Segmentkupplung zu längeren Peptidketten benötigt werden. Von besonderem Vorteil ist weiterhin, daß auf die empfindlichen, zum Teil gefährlichen Reagentien wie HF, TlOEt o.ä. verzichtet werden kann.

Zur Umeesterung der Säurederivate wird nach dem erfindungsgemäßen Verfahren üblicherweise wie folgt gearbeitet:

Der Ester wird in einem Alkohol evtl. unter Zusatz eines weiteren Lösungsmittels wie z.B. THF, CH₂Cl₂ o.a. gelöst oder suspendiert. Nach Zusatz der Amidinbase, z.B. DBU oder DBN, die in 0.01-10 molarer Menge, bevorzugt in 0.2-4 molarer Menge eingesetzt wird, und Zusatz der Metallverbindung, bevorzugt werden Salze des Magnesium oder Caesiums und besonders bevorzugt des Lithiums in 0,1-20 molarer Menge, insbesondere in 2-10 molarer Menge, wird bei Temperaturen zwischen -30°C und 120°C, bevorzugt bei Temperaturen zwischen -20°C und 65°C umgesetzt. Bei razemisierungsempfindlichen Estern wird bevorzugt zwischen -20°C und 30°C bei kurzen (gerade notwendigen) Reaktionszeiten umgesetzt.

Bei der Umesterung von Estern niederer Alkohole mit komplexeren Alkoholen kann es von Vorteil sein, den freigesetzten niederen Alkohol während der Reaktion destillativ zu entfernen.

Zur Verseifung der Säurederivate, bevorzugt ein Ester, wird nach dem erfindungsgemäßen Verfahren üblicherweise wie folgt gearbeitet:

Das Säurederivat wird in einem Lösungsmittel, bevorzugt werden Ether wie THF oder Dioxan eingesetzt, gelöst oder suspendiert. Nach Zusatz von Wasser (1 +, bevorzugt 10 - 100fach molarer Menge), der Amidinbase, z.B. DBU oder DBN, die in 1-10 molarer Menge, bevorzugt in 1-4 molarer Menge eingesetzt wird, und Zusatz der Metallverbindung, bevorzugt werden Salze des Lithiums, Magnesiums oder Caesiums in 0,1-20 molarer Menge, insbesonders in 2-10 molarer Menge, wird bei Temperaturen zwischen -20° und 65°C umgesetzt. Die Reihenfolge der Zugabe ist beliebig. Bei razemisierungsempfindlichen Derivaten wird bevorzugt zwischen -20° und 30°C bei kurzen Reaktionszeiten umgesetzt.

Als Metallverbindungen werden bei den obigen Verfahren Halogenide, insbesondere Bromid oder auch Chlorid, (insbesondere bei Verseifung) Hydroxid, Perchlorat, Acetat, Sulfat oder Carbonat bevorzugt. Bei Umesterungen - oder alkoholyse Reaktionen sind auch die Alkoholate der Metallverbindungen geeignet.

Zur Ammonolyse eines Carbonsäureesters, vorzugsweise eines Aminosäure- oder Peptidesters, wird der Ester in einem polaren Lösungsmittel, insbesondere THF oder Dioxan, dem etwas DMF (bis 30 vol-%) zugegeben sein kann, gelöst oder suspendiert. Die Amidinbase und die Metallverbindung, vorzugsweise ein Lithiumsalz, ein Palladiumsalz, eine Kupfer(I)verbindung, als Anion eignen sich Halogenide und Perchlorat besonders gut, werden hinzugegeben und NH₃ wird unter Kühlung eingeleitet. Besonders geeignete Metallverbindungen sind LiBr, LiClO₄, KF, CuCl, PdCl₂, wobei diese Salze, insbesondere das KF, auf Al₂O₃ in das Reaktionsgemisch eingebracht werden können.

Amidinbasen sind organische Verbindungen mit dem Strukturelement 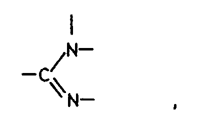 wobei die freien Valenzen der Stickstoffatome mit Wasserstoff und bevorzugt (insbesondere alle) mit Kohlenstoffatomen verbunden sind. Die freie Valenz am Kohlenstoffatom ist bevorzugt mit einem weiteren Kohlenstoffatom verbunden, aber z. B. auch ein weiteres Stickstoffatom ist möglich.

Als Amidinbase werden bevorzugt nicht nucleophile tertiäre Basen eingesetzt. Besonders bevorzugt sind die bicyclischen Verbindungen 1,8-Diazabicyclo[5.4.0.]undec-7-en (DBU) oder 1,5-Diazabicyclo[4.3.0]non-5-en (DBN). Die Amidinbase wird üblicherweise in einer 0,01 - 10 molaren Menge gegenüber dem Säurederivat eingesetzt, zu den besten Ergebnissen führten 0,2 - 4 molare Mengen. Da bei der Esterverseifung eine freie Säuregruppe entsteht, muß die Amidinbase hier in mindestens molarer Menge eingesetzt werden, wenn nicht die Säurefunktion durch eine weitere Hilfsbase, vorzugsweise ein tertiäres Amin wie z.B. Triethylamin, abgefangen wird.
Die Hilfsbase kann dabei auch in einem Puffersystem vorliegen.

Die Metallverbindung, besonders geeignet sind Lithium- und ferner Magnesium- oder Cäsiumsalze, wird üblicherweise in 0,1 - 20 molarer Menge eingesetzt. Besonders günstig sind 2 - 10 molare Mengen der Metallverbindung, jeweils bezogen auf das Säurederivat.

Bei den Untersuchungen, die zur vorliegenden Erfindung führten, zeigte es sich auch, daß in einigen Fällen allein Lithiumhydroxid, oder ein anderes Lithiumsalz und eine Base, so daß Lithium- und Hydroxidionen in der Reaktionslösung vorliegen, zur Verseifung von Aminosäure- oder Peptidestern eingesetzt werden kann siehe Stammanmeldung. Hierbei ist eine 1,0 - 20 molare Menge an Lithiumhydroxid geeignet, bevorzugt ist eine 2 - 20 molare Menge an Lithiumhydroxid gegenüber der zu verseifenden Verbindung. Liegt das Lithiumhydroxid in einem Puffersystem vor bzw. ist eine Hilfsbase zugegen, dann kann die Lithiumverbindung auch schon in 0,1 molarer Menge eingesetzt werden. Die entstehende freie Säure wird dann durch die Hilfsbase und das Puffersystem neutralisiert, so daß der alkalische Charakter der Reaktionslösung zur Verseifung erhalten bleibt.

Anhand nachfolgender Beispiele wird die Erfindung näher ausgeführt.

Allgemeine Vorschriften zur Umesterung von Carbonsäureestern
A: LiBr (5 eq.) und der entsprechende Carbonsäureester (1 eq.) werden unter trockenem Argon in einer geeigneten Menge des gewünschten absoluten Alkohols gelöst oder suspendiert, so daß eine 0,2 bis 0,3 M Konzentration erhalten wird. Frisch destilliertes DBU (0,5 eq.) werden hinzugegeben und die Lösung bei Raumtemperatur gerührt. Der Reaktionsverlauf wird mittels Dünnschichtchromatographie oder Gaschromatographie verfolgt. Sobald keine weitere Umsetzung mehr erfolgt, wird die Reaktionmischung am Rotationsverdampfer unter Vakuum konzentriert und mit einer gesättigten wässrigen NH₄Cl oder einer 1 N HCl-Lösung hydrolysiert. Das Produkt wird zweimal mit Diethylether ausgeschüttelt, die vereinigten organischen Fraktionen werden mit Salzlauge bis zur neutralen Reaktion gewaschen und dann über Na₂SO₄ getrocknet. Nach Entfernung des Lösungsmittels im Vakuum wird das Rohprodukt durch Destillation oder Blitzchromatographie gereinigt.
B: Bei teuren Alkoholen wird LiBr, der entsprechende Methylester und eine stöchiometrische oder leicht überstöchiometrische Menge des Alkohols (1 - 2 eq.) in einer Mischung aus Tetrahydrofuran/Methylenchlorid (3:1 v/v) entsprechend der Methode A aufgelöst. Die Reaktionsmischung wird dann unter trockenem Argon unter Rückfluß erhitzt, das freigesetzte Methanol wird in einem 5 A Molekularsieb abgefangen, der in einem Tropftrichter oder einem Extraktor zwischen dem Reaktionskolben und dem Rückflußkühler angeordnet ist. Der Verlauf der Reaktion wird wie unter Methode A verfolgt, die Aufarbeitung erfolgt entsprechend.

### Allgemeine Methode zur Aufarbeitung von Peptidestern

Die Reaktionsmischung wird zu 200 ml Ethylacetat (150 ml Ethylacetat in einem zweiten Scheidetrichter) gegeben, der Extrakt wird nacheinander mit 100 ml 1 N HCl, 50 ml 1 N HCl, 100 ml 1 M KHCO₃, 50 ml 1 M KHCO₃ und zweimal mit 50 ml H₂O gewaschen, über MgSO₄ getrocknet und im Vakuum eingedampft. Der Rückstand wird über mehrere Stunden unter reduziertem Druck getrocknet.

### Beispiel 1

### Umesterung von Phenylessigsäuremethylester zu Phenylessigsäureethylester

Entsprechend der Methode A werden Phenylessigsäuremethylester (4,51 g, 30 mmol) und LiBr (13,03 g, 150 mmol) in Ethanol (150 ml) gelöst. DBU (2,28 g, 15 mmol) wird hinzugegeben und die Reaktionsmischung bei Raumtemperatur 1 Stunde gerührt. Anschließend wird hydrolysiert und wie beschrieben aufgearbeitet. Vakuumdestillation ergab 4,40 g (90 % Ausbeute der Theorie) von reinem Phenylessigsäureethylester. Sdp. 65,5 - 66°C/1 Torr.

### Beispiel 2

### Umesterung von Phenylessigsäuremethylester zu Phenylessigsäure-(R)-menthylester

Entsprechend der Methode B werden Phenylessigsaüremethylester (751 mg, 5 mmol), LiBr (2,17 g, 25 mmol) und (R)-(-)-Menthol (751 mg, 5 mmol) in THF/CH₂Cl₂ (3:1 v/v, 20 ml) gelöst. DBU (0,37 ml, 2,5 mmol) wurde hinzugegeben und die Reaktionsmischung für mehrere Stunden unter Rückfluß gekocht. Das Dünnschichtchromatogramm (SiO₂; Pentan/Diethylether 4:1 v/v) zeigte, daß die Umesterung nach 24 Stunden Kochen unter Rückfluß nicht vollendet war. Trotzdem wurde die Reaktionsmischung hydrolysiert und aufgearbeitet. Blitzchromatographie (SiO₂; Pentan/Dietylether 4:1 v/v) ergab 691 mg (50 % Ausbeute) von Phenylessigsäure-(R)-menthylester als ein im ¹H-NMR im wesentlichen reines Öl.

### Beispiel 3

### Umesterung von Phenylessigsäuremethylester zu Phenylessigsäure-2-trimethylsilylethylester

Entsprechend Methode B wurde Phenylessigsäuremethylester (751 mg, 5 mmol) mit 2-Trimethylsilylethanol (1,18 g, 1,43 ml, 10 mmol) in THF/CH₂Cl₂ (3:1/v, 20 ml) unter Rückfluß umgeestert. Nach 8 Stunden Rückfluß wurde die Reaktionsmischung hydrolysiert und aufgearbeitet. Im GC und ¹H-NMR war das in quantitative Ausbeute erhaltene Rohprodukt im wesentlichen rein (≥ 99 % im GC).

### Beispiel 4

### Darstellung von rac-(4RS,5SR)-5-Isopropyl-2-oxazolidinon-4-carbonsäureethylester (3b)

rac-(4SR,5RS,8SR)-1-Aza-3,7-dioxa-4-(2'-propyl)-8-(tert.-butyl)-bicylo[3.3.0]-octan-2,6-dion (302 mg, 1,25 mmol) und LiBr (543 mg, 6,25 mmol) wurden in Ethanol (30 ml) gelöst. DBU (0,37 ml, 2,5 mmol) wurde hinzugefügt und die erhaltene Lösung bei Raumtemperatur für 2 Stunden gerührt. Nach saurer Hydrolyse und der üblichen Aufarbeitung, Blitzchromatographie (SiO₂; CH₂Cl₂/Ethylacetat 4:1 v/v), wurden 192 mg (Ausbeute 76 %) an 3b als ein farbloses, viskoses Öl erhalten.

### Beispiel 5

### Darstellung von Boc-Phe-Ala-OEt

Nach dem Lösen von Boc-Phe-Ala-OMe (701 mg, 2 mmol) und LiBr (869 mg, 10 mmol) in Ethanol (10 ml), wurde DBU (150 µl, 1 mmol) bei Raumtemperatur hinzugegeben. Nach 6 Minuten wurde die Reaktionslösung mit 1N HCl (3 ml) behandelt und wie oben beschrieben aufgearbeitet.
Ausbeute: 700 mg (96 %) mit ≡ 2 % Ausgangsprodukt (¹H-NMR) und einem Anteil an D-Ala von 4 % (GC).

### Beispiel 6

### Darstellung von Boc-Phe-Ala-OCHMe₂

Nach dem Auflösen von Boc-Phe-Ala-OMe (701 mg, 2 mmol) und LiBr (869 mg, 10 mmol) in Isopropanol (10 ml), wurde bei - 10°C DBU (150 µ, 1 mmol zugegeben. Nach Rühren über 44 Stunden bei dieser Temperatur wurde die Reaktionsmischung mit verdünntem HCl/Diethylether (3 ml) behandelt und wie oben beschrieben aufgearbeitet.
Ausbeute: 664 mg (88 %) mit 4 % Ausgangsprodukt (¹H-NMR) und einem Anteil an D-Ala von 4 % (GC)

### Beispiel 7

### Darstellung von Boc-Phe-Ala-OCH₂CH=CH₂(7d)

Nach Lösen von Boc-Phe-Ala-OMe (701 mg, 2 mmol) und LiBr (869 mg, 10 mmol) in Allylalkohol (10 ml), wurde bei 0°C DBU (150 µl, 1 mmol) zugegeben.
Nach 6 Stunden Rühren bei 0°C wurde die Reaktionsmischung mit verdünntem HCl/Diethylether (3 ml) versetzt und wie oben beschrieben aufgearbeitet.
Ausbeute: 686 mg (91 %) an schwach bräunlichem 7d mit 3 % Ausgangsprodukt (¹H-NMR) und einem Gehalt an D-Ala von 5 % (GC).

### Beispiel 8

### Peptid-Harz-Alkoholyse, Boc-Leu-Ala-Gly-Val-OMe (15b)

Nach Suspendieren von Boc-Leu-Ala-Gly-Val-(PS-Pam-Harz) (15a) (300 mg, 0,168 mmol Peptid) in 3 ml einer 0,28 M LiBr/Methanollösung (487 mg LiBr/20 ml Methanol) und Rühren bei Raumtemperatur über 15 Minuten wurde DBU (50 µl, 0,34 mmol) zugegeben. Nach Rühren bei Raumtemperatur über 4 Stunden wurde die Reaktionsmischung gefiltert, das Harz mit Ethylacetat (∼ 10 ml) gewaschen, mit 1N HCl (∼ 10 ml) behandelt und zweimal mit Ethylacetat (∼ 10 ml) extrahiert. Nach dem Trocknen der vereinigten organischen Extrakte mit Magnesiumsulfat, Filtern, Verdampfen des Lösungsmittels und Trocknen im Hochvakuum, wurden 78 mg an 15b erhalten, leicht verunreinigt mit einem D-Val Anteil von 1 % (GC). Weitere Reinigung durch Blitzchomatographie (5 % Methanol in Diethylether) ergab nach 24 stündiger Trocknung im Hochvakuum 66 mg (83 %) eines weißen Pulvers an 15b mit einem Schmelzpunkt von 71 - 72°C.

### Beispiel 9

### Peptid-Harz-Spaltung, Darstellung von Boc-Leu-Ala-Gly-Val-OH (15c)

Nach Suspendieren von Boc-Leu-Ala-Gly-Val-(PS-Pam-Harz) (15a) (150 mg, 0,093 mmol Peptid) in einer Lösung von LiBr (40 mg, 0,46 mmol) in THF (1,8 ml) und Wasser (0,2 ml) und Rühren über 15 Minuten bei Raumtemperatur, wurde DBU (7 µl, 0,047 mmol) hinzugegeben. Nach 4 stündigem Rühren bei Raumtemperatur wurde die Reaktionsmischung gefiltert, das Harz mit Ethylacetat (∼ 10 ml) gewaschen, mit 1N HCl (∼ 10 ml) behandelt und zweimal mit Ethylacetat (∼ 10 ml) extrahiert. Nach dem Trocknen der vereinigten organischen Extrakte mit MgSO₄, Filtern und Vertreiben des Lösungsmittels wurde im Hochvakuum getrocknet. Das erhaltene Produkt (81 mg) war leicht verunreinigt mit einem D-Val Anteil von 1 % (GC). Die Ausbeute wurde mittels 1^{H}-NMR am Rohprodukt mit Acetonitril als internem Standard bestimmt: 34 mg (81 %). Veresterung des Rohprodukts mit CH₂N₂ führte zu einem Produkt mit einem ¹H-NMR Spektrum entsprechend dem von 15b.

### Beispiel 10

### Peptid-Harz-Alkoholyse, Boc-Leu-Ala-Gly-Phe-OMe (16b)

Nach Suspension von Boc-Leu-Ala-Gly-Phe-(PS-Pam-Harz) (16a) (150 mg, 0,084 mmol Peptid) in einer Lösung von LiBr (36 mg, 0,41 mmol) in MeOH (2 ml) wurde nach 15-minütigem Rühren bei 0°C DBU (6,3 µl, 0,042 mmol) zugegeben. Nach 8-stündigem Rühren bei 0°C wurde die Reaktionsmischung gefiltert, das Harz mit Ethylacetat (ca. 10 ml) gewaschen, mit 1N HCl (ca. 10 ml) behandelt und wie oben (halbe Lösungsmittelmenge) aufgearbeitet. Erhalten wurden 64 mg eines farblosen Öls von 16b mit einem D-Phe Anteil von 2 % (GC). Der Gehalt an 16b wurde mittels 1^{H}-NMR am Rohprodukt mit Acetonitril als internem Standard ermittelt: 38 mg (86 %). Eine weitere Reinigung mit Blitzchromatographie (10 % v/v MeOH/Diethylether) ergab nach 24 Stunden Trocknen über Hochvakuum 36 mg (82 %) eines weißen Pulvers von 16b.

### Beispiel 11

### Peptid-Harz-Spaltung, Darstellung von Boc-Leu-Ala-Gly-Phe-OH (16c)

Nach Suspendieren von Boc-Leu-Ala-Gly-Phe-(PS-Pam-Harz) (16a) (150 mg, 0,084 mmol Peptid) in einer Lösung von LiBr (36 mg, 0,41 mmol) in THF/10 % v/v H₂O (2 ml) wurde nach 15-minütigem Rühren bei Raumtemperatur DBU (6,3 µl, 0,042 mmol) zugegeben. Nach 4 Stunden Rühren bei Raumtemperatur wurde die Reaktionsmischung gefiltert, das Harz mit Ethylacetat (ca. 10 ml) gewaschen, mit 1N HCl (ca. 10 ml) behandelt und zweimal mit Ethylacetat (ca. 10 ml) extrahiert. Nach Aufarbeitung wie oben wurden 96 mg an verunreinigtem 16c mit einem D-Phe Anteil von 2 % (GC) isoliert. Der Anteil an 16 c wurde über ¹H-NMR am Rohprodukt mit Acetonitril als interem Standard zu 40 mg (93 %) ermittelt. Veresterung des Rohprodukts mit CH₂N₂ ergab ein ¹H-NMR Spektrum entsprechend zu 16b.

### Beispiel 12

### Ac-D-Nal-D-p-Cl-Phe-D-Pal-Ser-Tyr-D-Cit-Leu-OH

250 mg (0,223 mmol) Ac-D-Nal-D-p-Cl-Phe-D-Pal-Ser-Tyr-D-Cit-Leu-OMe wurden in 15 ml THF suspendiert, die Suspension mit 1 ml Wasser und der Lösung von 11,2 mg (0,468 mmol) LiOH in 1 ml Wasser versetzt und die Reaktionsmischung 4 Stunden bei Raumtemperatur gerührt. Dannach war laut HPLC kein Edukt mehr vorhanden. Man brachte die Reaktionslösung mit 1N Salzsäure auf einen pH von 4, entfernte das THF im Vakuum, verdünnte den Rückstand mit 15 ml Wasser und saugte ab. Das Produkt wurde mit 30 ml Acetonitril bei 80°C heiß digeriert, erneut abgesaugt und getrocknet. Man erhielt schließlich 220 mg (90 %) Ac-D-Nal-D-p-Cl-Phe-D-Pal-Ser-Tyr-D-Cit-Leu-OH, laut HPLC mit einer Reinheit von 98,5 %. Im ¹H-NMR-Spektrum war das Signal des Methylesters bei 3,6 ppm nicht mehr vorhanden, ansonsten entsprach das Spektrum einem Vergleichsspektrum von auf unabhängigem Wege hergestellten Ac-D-Nal-D-p-Cl-Phe-D-Pal-Ser-Tyr-D-Cit-Leu-OH. Ein GC-Racemattest zeigte keine signifikante Racemisierung am Leucin (D-Leu 0,5 %).

### Beispiel 13

### Ammonolyse, Darstellung von Boc-Phe-Ala-NH₂

In 30 ml trockenem THF werden 300 mg (0,86 mmol) Boc-Phe-Ala-OMe und 274 mg (2,6 mmol, 3 eq) LiClO₄ gelöst sowie 400 mg KF auf Al₂O₃ (≙ 2,2 mmol F⁻) suspendiert. Bei 0°C läßt man getrocknetes NH₃-Gas durchströmen. Nach 24 h ist das Edukt weitgehend verbraucht. ¹H-NMR ergab einen Esteranteil von ca. 10 %; im DC konnten keine Nebenprodukte festgestellt werden.

## Patentansprüche

1. Verfahren zur Umsetzung eines Phosphorsäure-, Phosphonsäure- oder Carbonsäurederivates mit einem Alkohol, Wasser oder Ammoniak in Gegenwart einer Amidinbase und unter Zusatz einer Metallverbindung, ausgewählt aus Salzen von Lithium, Magnesium oder Caesium, oder bei Ausschluß von Wasser unter Zusatz eines Alkoholats von Lithium, Magnesium oder Caesium.

2. Verfahren nach Anspruch 1,
**dadurch gekennzeichnet,**
daß das Derivat ein Ester oder Amid ist.

3. Verfahren nach Anspruch 1 oder 2,
**dadurch gekennzeichnet,**
daß es in Anwesenheit eines zusätzlichen polaren organischen Lösungsmittels durchgeführt wird.

4. Verfahren nach einem der vorstehenden Ansprüche,
**dadurch gekennzeichnet,**
daß das Salz ein Halogenid, Perchlorat, Acetat, Sulfat oder Carbonat ist.

5. Verfahren nach einem der vorstehenden Ansprüche,
**dadurch gekennzeichnet,**
daß die Amidinbase in 0,01 bis 10 molarer Menge eingesetzt wird.

6. Verfahren nach einem der vorstehenden Ansprüche,
**dadurch gekennzeichnet,**
daß die Metallverbindung in 0,1 - 20 molarer Menge eingesetzt wird.

7. Verfahren nach einem der vorstehenden Ansprüche,
**dadurch gekennzeichnet,**
daß es bei Temperaturen zwischen -30 °C und +120 °C durchgeführt wird.

8. Verfahren nach einem der vorstehenden Ansprüche,
**dadurch gekennzeichnet,**
daß die Amidinbase ein Strukturelement der Formel 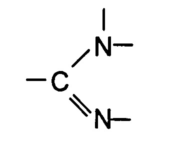 aufweist, in dem die freien Valenzen der Stickstoffatome mit Wasserstoff und Kohlenstoffatomen verbunden sind und die freie Valenz am Kohlenstoffatom mit einem weiteren Kohlenstoffatom oder einem weiteren Stickstoffatom verbunden ist.

9. Verfahren nach einem der vorstehenden Ansprüche,
**dadurch gekennzeichnet,**
daß das Derivat ein(e) über eine Ester- oder Amidbindung an ein Polymerträger gebundene(s) Peptid, Aminosäure oder Nucleinsäure ist.

10. Verfahren nach einem der vorstehenden Ansprüche,
**dadurch gekennzeichnet,**
daß zur Verseifung des Derivats mit Wasser die Amidinbase in mindestens molarer Menge eingesetzt wird, wenn nicht die Säurefunktion durch eine weitere Hilfsbase abgefangen wird.

11. Verfahren nach einem der vorstehenden Ansprüche 1 bis 8,
**dadurch gekennzeichnet,**
daß ein Aminosäureester oder ein Peptidester als Carbonsäurederivat in einem polaren Lösungsmittel, insbesondere Tetrahydrofuran oder Dioxan, mit mindestens einem Äquivalent NH₃ durch Ammonolyse zum Amid umgesetzt wird.

12. Verfahren nach Anspruch 10,
**dadurch gekennzeichnet,**
daß die Ammonolyse in einem Gemisch von Tetrahydrofuran und Dimethylformamid mittels eines Lithiumsalzes oder eines Palladiumsalzes oder einer Kupfer(I)-Verbindung anstelle von Magnesium- oder Caesiumsalz erfolgt, wobei das Anion Halogenid oder Perchlorat ist.

## Claims

1. A method of reacting a phosphoric acid, phosphonic acid or carboxylic acid derivative with an alcohol, water or ammonia in the presence of an amidine base and with the addition of a metal compound, selected from salts of lithium, magnesium or caesium or, if water is excluded, with the addition of an alcoholate of lithium, magnesium or caesium.

2. A method according to claim 1, characterised in that the derivative is an ester or amide.

3. A method according to claim 1 or claim 2, characterised in that it is carried out in the presence of an additional polar organic solvent.

4. A method according to any one of the preceding claims, characterised in that the salt is a halide, perchlorate, acetate, sulphate or carbonate.

5. A method according to any one of the preceding claims, characterised in that the amidine base is used in a 0.01 to 10 molar amount.

6. A method according to any one of the preceding claims, characterised in that the metal compound is used in a 0.1 to 20 molar amount.

7. A method according to any one of the preceding claims, characterised in that it is carried out at temperatures of between -30 °C and +120 °C.

8. A method according to any one of the preceding claims, characterised in that the amidine base comprises a structural element of the formula 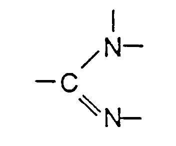 in which the free valencies of the nitrogen atoms are connected with hydrogen and carbon atoms and the free valency on the carbon atom is connected with a further carbon atom or a further nitrogen atom.

9. A method according to any one of the preceding claims, characterised in that the derivative is a peptide, amino acid or nucleic acid bonded to a polymer support by means of an ester or amide bond.

10. A method according to any one of the preceding claims, characterised in that, for the purpose of saponification of the derivative with water, the amidine base is used in at least molar amount, unless the acid function is scavenged by a further auxiliary base.

11. A method according to any one of preceding claims 1 to 8, characterised in that an amino acid ester or a peptide ester is reacted as a carboxylic acid derivative in a polar solvent, in particular tetrahydrofuran or dioxane, with at least an NH₃ equivalent by ammonolysis to form the amide.

12. A method according to claim 10, characterised in that the ammonolysis is carried out in a mixture of tetrahydrofuran and dimethyl formamide by means of a lithium salt or a palladium salt or a copper(I) compound instead of magnesium or caesium salt, wherein the anion is halide or perchlorate.

## Revendications

1. Procédé de transformation d'un dérivé d'acide phosphorique, phosphonique ou carboxylique avec un alcool, l'eau ou l'ammoniac en présence d'une base amidine, et en ajoutant un composé métallique, choisi parmi les sels de lithium, de magnésium ou de césium ou bien à l'abri de l'eau en ajoutant un alcoolate de lithium, de magnésium ou de césium.

2. Procédé selon la revendication 1,
caractérisé en ce que
le dérivé est un ester ou un amide.

3. Procédé selon la revendication 1 ou 2,
caractérisé en ce qu'
on procède en présence d'un solvant organique supplémentaire polaire.

4. Procédé selon l'une des revendications précédentes,
caractérisé en ce que
le sel est un halogénure, un perchlorate, un acétate, un sulfate ou un carbonate.

5. Procédé selon l'une des revendications précédentes,
caractérisé en ce qu'
on met en oeuvre la base amidine en quantités 0,01 à 10 molaires.

6. Procédé selon l'une des revendications précédentes,
caractérisé en ce qu'
on met en oeuvre le composé métallique en quantités 0,1 à 20 molaires.

7. Procédé selon l'une des revendications précédentes,
caractérisé en ce qu'
on l'effectue à des températures comprises entre -30°C et + 120°C.

8. Procédé selon l'une des revendications précédentes,
caractérisé en ce que
la base amidine possède un élément de structure de formule : 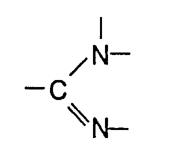 dans lequel les valences libres de l'atome d'azote sont liées à l'hydrogène et à des atomes de carbone et la valence libre à l'atome de carbone, est liée à un autre atome de carbone ou à un autre atome d'azote.

9. Procédé selon l'une des revendications précédentes,
caractérisé en ce que
le dérivé est un peptide, un aminoacide ou un acide nucléique fixé par l'intermédiaire d'une liaison ester ou amide sur un support polymère.

10. Procédé selon l'une des revendications précédentes,
caractérisé en ce que
pour la saponification du dérivé avec l'eau, on met en oeuvre la base amidine dans des quantités au moins molaires lorsque la fonction acide n'est pas captée par une autre base auxiliaire.

11. Procédé selon l'une des revendications précédentes,
caractérisé en ce qu'
on fait réagir un ester d'aminoacide ou un ester de peptide comme dérivé d'acide carboxylique dans un solvant polaire, en particulier le tétrahydrofuranne ou le Dioxanne, avec au moins un équivalent de NH₃ par ammonolyse, en amide.

12. Procédé selon la revendication 10,
caractérisé en ce que
l'ammonolyse s'effectue dans un mélange de tétrahydrofuranne et de diméthylformamide à l'aide d'un sel de lithium ou d'un sel de palladium ou d'un composé du cuivre (I) au lieu d'un sel de magnésium ou de césium, pour lesquels l'anion est un halogénure ou un perchlorate.
